# EUROPÄISCHE PATENTANMELDUNG

(11) **EP 4 666 977 A1**
(43) Veröffentlichungstag der Anmeldung: **24.12.2025**
(21) Anmeldenummer: 25183114.5
(22) Anmeldetag: 16.06.2025
(51) Int. Cl.: A61B 34/20, A61B 90/00, F16C 11/06, F16C 11/10

(54) **VORRICHTUNG ZUM HALTEN EINER MIT EINER BILD- ODER SIGNALERFASSUNGSEINRICHTUNG EINES OP-NAVIGATIONSSYSTEMS ZUSAMMENWIRKENDEN SENDERANORDNUNG**

(30) Priorität: 19.06.2024 DE 102024117246
(71) Anmelder: Aesculap AG, 78532 Tuttlingen (DE)
(72) Erfinder: Firmbach, Franz-Peter, 78576 Emmingen-Liptingen (DE); Welte, Katharina, 78056 Villingen-Schwenningen (DE); Nonnenmann, Martin, 78573 Wurmlingen (DE)
(74) Vertreter: DREISS Patentanwälte PartG mbB

(57) **Zusammenfassung**

Die Erfindung betrifft eine Vorrichtung (2) zum Halten einer mit einer Bild- oder Signalerfassungseinrichtung (4) eines OP-Navigationssystems zusammenwirkenden Senderanordnung (6) in einer OP-Situation am Patienten, umfassend ein Basisteil (16) und wenigstens ein Befestigungsmittel (18), welches in einen Knochen (8) des Patienten einzubringen ist, um das Basisteil (16) in ortsfester Orientierung zum Knochen (8) während der OP-Situation anzuordnen, und eine an dem Basisteil gehaltene Gelenkanordnung (21); erfindungsgemäß wird vorgeschlagen, dass die Gelenkanordnung (21) mit einem von der Grundgeometrie her kugelförmigen Kopf (30) und einem sich davon wegerstreckenden und die Senderanordnung (6) tragenden Schaft (32) und mit einem zweikomponentigen Aufnahmeteil (24) für den Kopf (30) ausgebildet ist, bezüglich dessen der Kopf (30) und damit die Senderanordnung (6) in einer gewünschten Orientierung zum Knochen (8) festlegbar ist, und dass der Kopf (30) an seiner Oberfläche (39) nach radial außen vorstehende insbesondere verrundete Vorsprünge oder nach radial innen eintauchende insbesondere kalottenförmige Vertiefungen (48) oder ebene Facettenabschnitte (42) aufweist und dass das Aufnahmeteil (24) für den Kopf (30) einen hierzu zumindest bereichsweise derart formkomplementär ausgebildeten Innenabschnitt (38) aufweist, dass der Kopf (30) in einer diskreten auswählbaren Orientierung zu dem Aufnahmeteil (24) festlegbar ist, wobei die Vorsprünge, die Vertiefungen (48) oder die Facettenabschnitte (42) derart über die Oberfläche (39) des Kopfs (30) verteilt ausgebildet sind, dass nebeneinander befindliche diskrete auswählbare Orientierungen einen Winkel gemessen vom Kugelmittelpunkt von höchstens 15° zueinander einschließen.

## Beschreibung

Die Erfindung betrifft eine Vorrichtung zum Halten einer mit einer Bild- oder Signalerfassungseinrichtung eines OP-Navigationssystems zusammenwirkenden aktiven oder passiven Senderanordnung in einer OP-Situation am Patienten, umfassend ein Basisteil und wenigstens ein Befestigungsmittel, welches in einen Knochen des Patienten, insbesondere in einen Oberschenkel- oder Unterschenkelknochen bei einer endoprothetischen Hüft- oder Kniegelenksersatzversorgung, einzubringen ist, um das Basisteil in ortsfester Orientierung zum Knochen während der OP-Situation anzuordnen, und eine an dem Basisteil gehaltene Gelenkanordnung.

Eine derartige Vorrichtung ist durch die Anmelderin bekanntgeworden, wobei die Gelenkanordnung um zwei Achsen verschwenkbar und festlegbar ist. Um die nach oben stehende Achse herum sind aber nur vier Drehstellungen realisierbar.

Der vorliegenden Erfindung liegt die Aufgabe zugrunde, eine Vorrichtung der vorausgehend geschilderten Art dahingehend weiter zu verbessern, dass sie sich auf kostengünstige Art und Weise herstellen lässt, und dass der/die Chirurg/in in der OP-Situation eine noch weitergehende Freiheit bei der Anordnung des Basisteils der Vorrichtung am Knochen des Patienten zur Verfügung hat, wobei die Vorrichtung darüber hinaus einfach und betriebssicher bedienbar sein soll.

Diese Aufgabe wird bei einer Vorrichtung der genannten Art erfindungsgemäß dadurch gelöst, dass die Gelenkanordnung mit einem von der Grundgeometrie her kugelförmigen Kopf und einem sich davon wegerstreckenden und die Senderanordnung tragenden Schaft und mit einem zweikomponentigen Aufnahmeteil für den Kopf ausgebildet ist, bezüglich dessen der Kopf und damit die Senderanordnung in einer gewünschten Orientierung zum Knochen festlegbar ist, und dass der Kopf an seiner Oberfläche nach radial außen vorstehende insbesondere verrundete Vorsprünge oder nach radial innen eintauchende insbesondere kalottenförmige Vertiefungen oder ebene Facettenabschnitte aufweist und dass das Aufnahmeteil für den Kopf einen hierzu zumindest bereichsweise derart formkomplementär ausgebildeten Innenabschnitt aufweist, dass der Kopf in einer diskreten auswählbaren Orientierung zu dem Aufnahmeteil festlegbar ist, wobei die Vorsprünge, die Vertiefungen oder die Facettenabschnitte derart über die Oberfläche des Kopfs verteilt ausgebildet sind, dass nebeneinander befindliche diskrete auswählbare Orientierungen einen Winkel gemessen vom Kugelmittelpunkt von höchstens 15° zueinander einschließen.

Es soll also bei der Gelenkanordnung keine exakt kugelförmige Oberflächengeometrie mit konzentrischer Klemmung angestrebt werden, da die Anmelderin hierin eine zu geringe Sicherheit hinsichtlich der nach einmaliger Fixierung nicht erwünschten unbeabsichtigten Verstellbarkeit sieht. Insbesondere bei eher geringer Klemmkraft kann dann nämlich nicht sichergestellt werden, dass sich die eingestellte gewünschte Orientierung nicht wieder verstellt. Stattdessen soll eine im Ergebnis formschlüssig wirkende Fixierung zwischen dem Kopf und dem zweikomponentigen Aufnahmeteil der Gelenkanordnung eingesetzt werden, um eine spielfreie Orientierung und Festlegung der Vorrichtung zu realisieren. Dadurch, dass die diskreten auswählbaren Orientierungen einen Winkel von höchstens 15°, insbesondere von höchstens 12° und vorzugsweise von höchstens 10° aufweisen, kann sogleich eine große Freiheit bei der Positionierung des Basisteils am Knochen erzielt werden, um den Kopf mit der Senderanordnung so ausrichten zu können, dass eine Ebene, in der sich die mehreren Sender befinden, ungefähr orthogonal zur Hauptachse der Bilderfassungseinrichtung orientiert ist.

Bei dem wenigstens einen Befestigungsmittel kann es sich um einen Anker, eine Schraube oder einen Gewindestift handeln, der bzw. die in einer Klemmaufnahme am Basisteil ortsfest fixierbar ist, so dass nach Einbringen des Befestigungsmittels in den Knochen des Patienten das Basisteil daran ortsfest fixierbar ist.

Weiter erweist es sich als vorteilhaft, dass das wenigstens eine Befestigungsmittel eine solche Länge hat, dass das Basisteil in einem Abstand von 5,0 - 50 mm von der Knochenoberfläche anordenbar ist.

Wie schon angedeutet, erweist es sich als vorteilhaft, wenn die Senderanordnung wenigstens drei, insbesondere vier Sender trägt, die auf sich kreuzenden Schenkeln in einer Ebene angeordnet sind, wobei ein Schenkel von dem sich von dem Kopf wegerstreckenden Schaft gebildet oder daran unmittelbar oder mittelbar befestigbar ist.

Zur formschlüssigen Fixierung des Kopfs bezüglich des Aufnahmeteils ist es nicht erforderlich, dass das mehrkomponentige Aufnahmeteil den Kopf gewissermaßen vollumfänglich umschließt, was ja auch eine Einschränkung für die Verschwenkbarkeit mit dem daran angeordneten Schaft mit sich bringen würde. Vielmehr erweist es sich als vorteilhaft und hinreichend, wenn das Aufnahmeteil in einer den Schaft einschließenden Schnittebene betrachtet den Kopf über einen Winkel von wenigstens 120°, insbesondere von wenigstens 130°, insbesondere von wenigstens 140°, insbesondere von höchstens 170°, insbesondere von höchstens 160°, insbesondere von höchstens 150°, umfängt und in einer orthogonal zu dem Schaft erstreckten Schnittebene betrachtet den Kopf über einen Winkel von wenigstens 30°, insbesondere von wenigstens 40° und von höchstens 90°, insbesondere von höchstens 80°, insbesondere von höchstens 70°, insbesondere von höchstens 60° umfängt.

Prinzipiell ist es auch denkbar und hinreichend, wenn das Aufnahmeteil oder jede Komponente des Aufnahmeteils nur einen formkomplementär ausgebildeten Innenabschnittsbereich aufweist, welcher gewissermaßen mit Vorsprüngen, Vertiefungen oder Facettenabschnitten des Kopfs in eine formschlüssig und kraftschlüssig wirkende Klemmverbindung oder Fixierverbindung gelangen kann. Es wäre aber auch denkbar, dass die gesamte Innenseite der jeweiligen Komponente des Aufnahmeteils formkomplementär zu der Oberfläche des Kopfs ausgebildet ist.

Weiter kann vorgesehen sein, dass die Komponenten des Aufnahmeteils gegeneinander festziehbar sind, und zwar insbesondere mittels einer Schraubverbindung, eines Knebel- oder Kniehebelmechanismus, wobei sich weiter als vorteilhaft erweist, wenn die Komponenten werkzeuglos, also rein durch manuelle Betätigung, gegeneinander festziehbar wieder voneinander lösbar sind.

Nach einer Ausführungsvariante der Erfindung weist der Kopf an seiner Oberfläche Vertiefungen auf, wobei die Vertiefungen insbesondere zylindrisch, konisch oder kalottenförmig sein können. Entsprechend ist bei dem Innenabschnitt des Aufnahmeteils wenigstens ein Vorsprung vorgesehen, welcher ganz oder teilweise in eine ihm gegenüber positionierte Vertiefung des Kopfs eingreifen kann, wenn die Komponenten des Aufnahmeteils unter Einschluss des Kopfs gegeneinander gezwungen oder festgezogen werden.

Hierfür kann in vorteilhafter Weise eine Schraubverbindung, ein Knebelmechanismus oder Kniehebelmechanismus verwendet werden.

Nach einer weiteren Ausführungsform der Erfindung erweist es sich als vorteilhaft, dass der Kopf an seiner Oberfläche Vertiefungen aufweist, wobei die Vertiefungen bei dem Kopf zylindrisch, konisch oder kalottenförmig ausgebildet sein können, und dass bei dem Innenabschnitt des Aufnahmeteils wenigstens ein Vorsprung mit einem gekrümmten, insbesondere kugelkopfförmig gekrümmten freien Ende ausgebildet ist und dass ein Krümmungsradius des kugelkopfförmig gekrümmten freien Endes größer ist als ein Radius der Vertiefungen im Bereich der Oberfläche des Kopfs. Dadurch, dass also die Nenngröße des freien Endes des Vorsprungs des Aufnahmeteils größer ist als das Nennmaß der Vertiefungen an der Oberfläche des Kopfs, lässt sich eine Selbstzentrierung zwischen Kopf und Aufnahmeteil herbeiführen, die sich bei der praktischen Verwendung als angenehm und daher vorteilhaft erweist.

Nach einer weiteren Ausführungsform kann vorgesehen sein, dass bei dem Innenabschnitt des Aufnahmeteils nach radial innen kraftbeaufschlagbare Stellstifte vorstehen, die in dem Aufnahmeteil verschieblich sind und jeweils in eine nach radial innen eintauchende insbesondere kalottenförmige Vertiefung bei dem Kopf zumindest teilweise eintauchen können, um den Kopf in einer diskreten auswählbaren Orientierung zu dem Aufnahmeteil zu fixieren. Die Verwendung kraftbeaufschlagbarer Stellstifte, insbesondere federkraftbeaufschlagbarer Stellstifte, ermöglicht bei verrundeter Ausbildung der Stellstifte eine angenehme Bedienbarkeit bei der Verstellung oder Einstellung des Kopfs in dem Aufnahmeteil.

Auch hier kann es sich als vorteilhaft erweisen, dass die Stellstifte ein gekrümmtes, insbesondere kugelkopfförmig gekrümmtes freies Ende aufweisen und dass ein Krümmungsradius des gekrümmten freien Endes größer ist als ein Radius der Vertiefungen im Bereich der Oberfläche des Kopfs.

Nach einer weiteren als besonders vorteilhaft zu bezeichnenden Weiterentwicklung wird vorgeschlagen, dass das zweikomponentige Aufnahmeteil vorzugsweise in jeder der Komponenten nach innen ausmündene Spülkanäle aufweist, um einen Zwischenraum zwischen Aufnahmeteil und Kopf durch Einleitung von Spülflüssigkeit außerhalb der OP-Situation reinigen zu können.

Weitere Merkmale, Einzelheiten und Vorteile der Erfindung ergeben sich aus den beigefügten Patentansprüchen und der zeichnerischen Darstellung und nachfolgenden Beschreibung bevorzugter Ausführungsformen der erfindungsgemäßen Vorrichtung.

In der Zeichnung zeigt:
- Figur 1: eine schematische Darstellung einer erfindungsgemäßen Vorrichtung zum Halten einer Senderanordnung in einer OP-Situation am Patienten;
- Figur 2: eine Gelenkanordnung der Vorrichtung nach Figur 1 in perspektivischer Darstellung;
- Figur 3: eine perspektivische Ansicht einer Komponente eines Aufnahmeteils der Gelenkanordnung nach Figur 2;
- Figur 4: eine perspektivische Ansicht eines Kopfs der Gelenkanordnung nach Figur 2 mit ebenen Facettenabschnitten;
- Figur 5: eine perspektivische Darstellung einer weiteren Ausführungsform eines Kopfs einer weiteren Gelenkanordnung;
- Figur 6: eine schematische Darstellung einer Ausführungsform einer Gelenkanordnung; und
- Figur 7: verdeutlicht schematisch unterschiedliche Nennmaße bei teilweise ineinandergreifenden Vorsprüngen und Vertiefungen bei einem Aufnahmeteil und einer Kugel einer Gelenkanordnung.

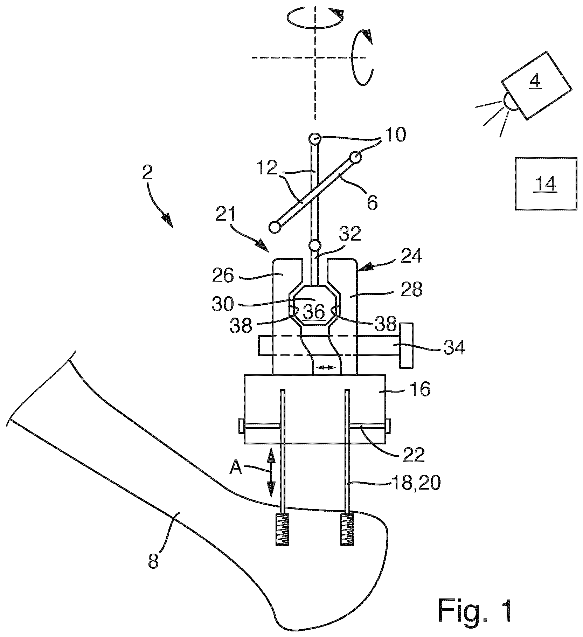

Figur 1 zeigt eine schematische Darstellung einer insgesamt mit dem Bezugszeichen 2 bezeichneten Vorrichtung zum Halten einer mit einer Bild- oder Signalerfassungseinrichtung 4 zusammenwirkenden Senderanordnung 6 in einer OP-Situation am Patienten, und zwar an einem zu versorgenden Knochen 8 des Patienten. Die Senderanordnung 6 umfasst vorzugsweise eine Mehrzahl von aktiven oder passiven Sendern 10, die arrayartig in einer Ebene angeordnet sind, die vorliegend von zwei Schenkeln 12 der Senderanordnung 6 gebildet wird, an denen die Sender 10 fixiert sind. Von den Sendern 10 ausgehende oder von ihnen reflektierte Signale werden von der Bild- oder Signalerfassungseinrichtung 4 erfasst und in einer Rechenvorrichtung des OP-Navigationssystems weiter verarbeitet, woraufhin von einem Display, das schematisch mit dem Bezugszeichen 14 bezeichnet ist, Auswertungen, Handlungsvorschläge und dergleichen Informationen für den Chirurgen angezeigt werden.

Die Vorrichtung 2 umfasst ein schematisch dargestelltes Basisteil 16, welches mittels beispielhaft zwei Befestigungsmitteln 18 in Form von Gewindestiften 20, am Knochen 8 des Patienten in einem wählbaren Abstand zum Knochen fixierbar ist. Dies erfolgt mittels einer jeweiligen Klemmschraube 22, welche das Basisteil 16 gegenüber dem betreffenden Gewindestift 20 fixiert.

Die Vorrichtung 2 umfasst weiter eine Gelenkanordnung 21 mit einem zweikomponentigen Aufnahmeteil 24, dessen beide Komponenten 26, 28 jeweils eine Art Klemmbacke für einen von der Grundgeometrie her kugelförmigen Kopf 30 bilden. Von dem Kopf 30 erstreckt sich ein Schaft 32 weg, der in der schematischen Darstellung übergeht in einen Schenkel 12 der Senderanordnung 6. Die beiden Komponenten 26, 28 des Aufnahmeteils 24 lassen sich mittels einer Gewindespindel 34 aufeinander zu bzw. voneinander weg bewegen, so dass der Kopf 30 in dem Aufnahmeteil 24 in Klemmstellung gebracht bzw. wieder gelöst werden kann.

Der in Figur 1 lediglich schematisch dargestellte Kopf 30 weist an seiner Oberfläche 36 nach radial außen vorstehende insbesondere verrundete Vorsprünge oder nach radial innen eintauchende insbesondere kalottenförmige Vertiefungen oder ebene Facettenabschnitte auf. Je nach Ausbildung umfassen die eine und/oder die andere Komponente 26, 28 des Aufnahmeteils 24 einen hierzu zumindest bereichsweise derart formkomplementär ausgebildeten Innenabschnitt 38, dass der Kopf 30 in einer Vielzahl von diskreten auswählbaren Orientierungen zu dem Aufnahmeteil 24 festlegbar ist. Nebeneinander befindliche diskret auswählbare Orientierungen schließen dabei einen Winkel gemessen vom Kugelmittelpunkt von höchstens 15° zueinander ein.

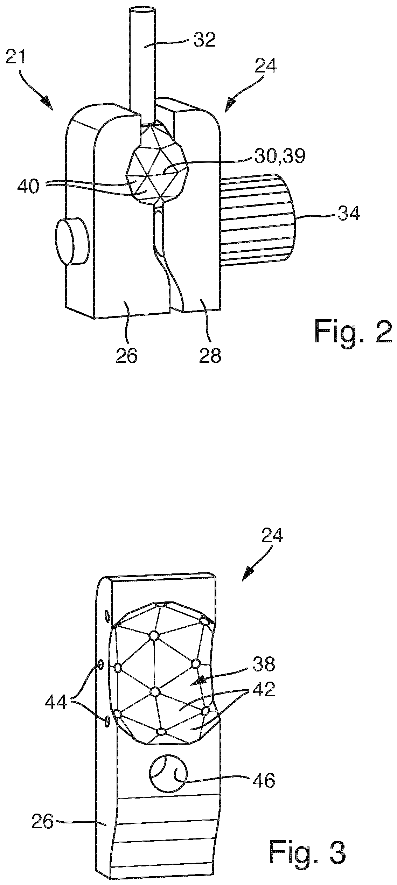

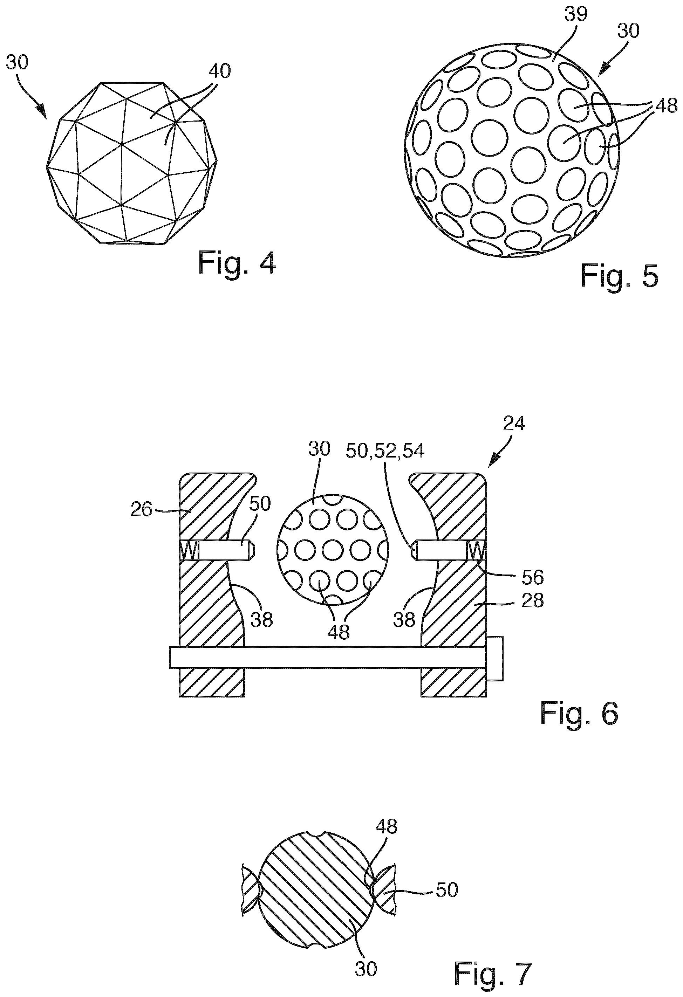

Figur 2 verdeutlicht eine erste Ausführungsform der erfindungsgemäßen Vorrichtung 2 in lediglich schematischer Darstellung der Gelenkanordnung 21 mit dem zweikomponentigen Aufnahmeteil 24, dem Kopf 30 mit zur Senderanordnung 6 führendem Schaft 32. Bei dieser Ausführungsform umfasst der Kopf 30 an seiner Oberfläche 39 ebene Facettenabschnitte 40. Die beiden Komponenten 26, 28 des Aufnahmeteils 24 umfassen einen jeweiligen formkomplementär hierzu ausgebildeten Innenabschnitt 38, der in Aufsicht in Figur 3 bei der Komponente 26 dargestellt ist. Man erkennt, dass auch bei dem Innenabschnitt zu den Facettenabschnitten 40 formkomplementär ausgebildete und angeordnete Facettenabschnitte 42 ausgebildet sind. Zusätzlich ist hier eine Ausführungsvariante verwirklicht und angedeutet, nach der Spülkanäle 44 in dem Aufnahmeteil 24 ausgebildet sind, die an dem Innenabschnitt 38 des Aufnahmeteils 24 münden. Auf diese Weise kann ein Zwischenraum zwischen dem Aufnahmeteil 24 und dem Kopf 30 gespült werden. Des Weiteren verdeutlichen die Figuren 2 und 3 die Stellschraube oder Gewindespindel 34 bzw. eine Ausnehmung 46 hierfür in dem Aufnahmeteil 24. Figur 4 verdeutlicht nochmal den Kopf 30 mit seinen ebenen Facettenabschnitten 40.

Figur 5 verdeutlicht ein anderes Ausführungsbeispiel der erfindungsgemäßen Vorrichtung, bei dem der Kopf 30 mit nach radial innen eintauchenden kalottenförmigen Vertiefungen 48 ausgebildet ist, die an die Oberflächenstruktur eines Golfballs erinnern. Die Vertiefungen 48 sind im beispielhaft dargestellten Fall kugelkalottenförmig, was aber nicht zwingend ist. Um eine formschlüssige und klemmschlüssige Fixierung des Kopfs 30 in einem in Figur 6 lediglich schematisch dargestellten Aufnahmeteil 24 zu realisieren, ist bei dem jeweiligen Innenabschnitt 38 der Komponenten 26, 28 des Aufnahmeteils 24 ein nach radial innen erstreckter Vorsprung 50 vorgesehen, welcher bei entsprechender Positionierung des Kopfs 30 in die Vertiefungen 48 bei der Oberfläche 39 des Kopfs 30 einzugreifen vermag. Die Vorsprünge 50 sind im beispielhaft dargestellten Fall von freien Enden 52 von kraftbeaufschlagbaren Stiften 54 gebildet, was schematisch durch die Darstellung eines Federelements 56 angedeutet ist.

Schließlich verdeutlicht Figur 7 wiederum schematisch, dass ein Nennmaß oder Krümmungsradius von nach innen erstreckten Vorsprüngen 50 bei dem Aufnahmeteil 24 größer sein kann als ein Nennmaß von nach innen eintauchenden Vertiefungen 48 bei dem Kopf 30. Hierdurch lässt sich ein selbstzentrierender Effekt erreichen.

Die hier beschriebene erfindungsgemäße Vorrichtung 2 in ihren verschiedenen Ausführungsvarianten erweist es sich als bedienungsfreundlich und lässt sich in wenig störanfälliger Weise in der OP-Situation einsetzen. Durch die Anordnung der formschlüssig miteinander zusammenwirkenden Vertiefungen, Vorsprüngen, oder Facettenabschnitten bei der Kugel einerseits und der bereichsweise formkomplementären Ausbildung bei dem Innenabschnitt der Komponenten des Aufnahmeteils andererseits lässt sich eine Vielzahl von diskret auswählbaren Orientierungen der Kugel und damit der Senderanordnung auswählen und fixieren, so dass eine zwar nicht stufenfreie, jedoch in einem engen Raster zur Verfügung stehende Orientierung ausgewählt werden kann.

## Patentansprüche

1. Vorrichtung (2) zum Halten einer mit einer Bild- oder Signalerfassungseinrichtung (4) eines OP-Navigationssystems zusammenwirkenden Senderanordnung (6) in einer OP-Situation am Patienten, umfassend ein Basisteil (16) und wenigstens ein Befestigungsmittel (18), welches in einen Knochen (8) des Patienten, insbesondere in einen Oberschenkel- oder Unterschenkelknochen bei einer endoprothetischen Hüft- oder Kniegelenksersatzversorgung, einzubringen ist, um das Basisteil (16) in ortsfester Orientierung zum Knochen (8) während der OP-Situation anzuordnen, und eine an dem Basisteil gehaltene Gelenkanordnung (21), **dadurch gekennzeichnet, dass** die Gelenkanordnung (21) mit einem von der Grundgeometrie her kugelförmigen Kopf (30) und einem sich davon wegerstreckenden und die Senderanordnung (6) tragenden Schaft (32) und mit einem zweikomponentigen Aufnahmeteil (24) für den Kopf (30) ausgebildet ist, bezüglich dessen der Kopf (30) und damit die Senderanordnung (6) in einer gewünschten Orientierung zum Knochen (8) festlegbar ist, und dass der Kopf (30) an seiner Oberfläche (39) nach radial außen vorstehende insbesondere verrundete Vorsprünge oder nach radial innen eintauchende insbesondere kalottenförmige Vertiefungen (48) oder ebene Facettenabschnitte (42) aufweist und dass das Aufnahmeteil (24) für den Kopf (30) einen hierzu zumindest bereichsweise derart formkomplementär ausgebildeten Innenabschnitt (38) aufweist, dass der Kopf (30) in einer diskreten auswählbaren Orientierung zu dem Aufnahmeteil (24) festlegbar ist, wobei die Vorsprünge, die Vertiefungen (48) oder die Facettenabschnitte (42) derart über die Oberfläche (39) des Kopfs (30) verteilt ausgebildet sind, dass nebeneinander befindliche diskrete auswählbare Orientierungen einen Winkel gemessen vom Kugelmittelpunkt von höchstens 15° zueinander einschließen.

2. Vorrichtung (2) nach Anspruch 1, **dadurch gekennzeichnet, dass** nebeneinander befindliche diskrete auswählbare Orientierungen einen Winkel gemessen vom Kugelmittelpunkt von höchstens 12°, insbesondere von höchstens 10° zueinander einschließen.

3. Vorrichtung (2) nach Anspruch 1 oder 2, **dadurch gekennzeichnet, dass** das wenigstens eine Befestigungsmittel (18) ein Anker, eine Schraube oder ein Gewindestift (20) ist, der bzw. die in einer Klemmaufnahme am Basisteil (16) ortsfest fixierbar ist, so dass nach Einbringen des Befestigungsmittels (18) in den Knochen (8) das Basisteil (16) daran ortsfest fixierbar ist.

4. Vorrichtung (2) nach Anspruch 1, 2 oder 3, **dadurch gekennzeichnet, dass** das wenigstens eine Befestigungsmittel (18) eine solche Länge hat, dass das Basisteil (16) in einem Abstand von 5,0 - 50 mm von der Knochenoberfläche anordenbar ist.

5. Vorrichtung (2) nach einem oder mehreren der vorstehenden Ansprüche, **dadurch gekennzeichnet, dass** die Senderanordnung (6) wenigstens drei, insbesondere vier Sender trägt, die auf sich kreuzenden Schenkeln (12) in einer Ebene angeordnet sind, wobei ein Schenkel (12) von dem sich von dem Kopf (30) wegerstreckenden Schaft (32) gebildet oder daran unmittelbar oder mittelbar befestigbar ist.

6. Vorrichtung (2) nach einem oder mehreren der vorstehenden Ansprüche, **dadurch gekennzeichnet, dass** das Aufnahmeteil (16) in einer den Schaft (32) einschließenden Schnittebene betrachtet den Kopf (30) über einen Winkel von wenigstens 120°, insbesondere von wenigstens 130°, insbesondere von wenigstens 140°, insbesondere von höchstens 170°, insbesondere von höchstens 160°, insbesondere von höchstens 150°, umfängt und in einer orthogonal zu dem Schaft (32) erstreckten Schnittebene betrachtet den Kopf (30) über einen Winkel von wenigstens 30°, insbesondere von wenigstens 40° und von höchstens 90°, insbesondere von höchstens 80°, insbesondere von höchstens 70°, insbesondere von höchstens 60° umfängt.

7. Vorrichtung (2) nach einem oder mehreren der vorstehenden Ansprüche, **dadurch gekennzeichnet, dass** die Komponenten (26, 28) des Aufnahmeteils (24) gegeneinander festziehbar sind, insbesondere mittels einer Schraubverbindung, eines Knebel- oder Kniehebelmechanismus.

8. Vorrichtung (2) nach einem oder mehreren der vorstehenden Ansprüche, **dadurch gekennzeichnet, dass** der Kopf (30) an seiner Oberfläche (39) Vertiefungen (48) aufweist und dass die Vertiefungen (48) bei dem Kopf (30) insbesondere zylindrisch, konisch oder kalottenförmig sind und dass bei dem Innenabschnitt (38) des Aufnahmeteils (24) wenigstens ein Vorsprung (50) vorgesehen, welcher ganz oder teilweise in eine ihm gegenüber positionierte Vertiefung (48) des Kopfs (30) eingreifen kann wenn die Komponenten (26, 28) des Aufnahmeteils (24) unter Einschluss des Kopfs (30) gegeneinander gezwungen oder festgezogen werden.

9. Vorrichtung (2) nach Anspruch 8, **dadurch gekennzeichnet, dass** der wenigstens eine Vorsprung (50) mit einem gekrümmten, insbesondere kugelkopfförmig gekrümmten freien Ende (52) ausgebildet ist und dass ein Krümmungsradius des kugelkopfförmig gekrümmten freien Endes größer (52) ist als ein Radius der Vertiefungen (48) im Bereich der Oberfläche des Kopfs (30).

10. Vorrichtung (2) nach einem oder mehreren der vorstehenden Ansprüche, **dadurch gekennzeichnet, dass** bei dem Innenabschnitt (38) des Aufnahmeteils (24) nach radial innen kraftbeaufschlagbare Stellstifte (54) vorstehen, die in dem Aufnahmeteil (24) verschieblich sind und die jeweils in eine nach radial innen eintauchende, insbesondere kalottenförmige Vertiefung (48) bei dem Kopf (30) zumindest teilweise eintauchen können, um den Kopf (30) in einer diskreten auswählbaren Orientierung zu dem Aufnahmeteil (24) zu fixieren.

11. Vorrichtung (2) nach Anspruch 10, **dadurch gekennzeichnet, dass** die Stellstifte (54) ein gekrümmtes, insbesondere kugelkopfförmig gekrümmtes freies Ende (52) aufweisen und dass ein Krümmungsradius des gekrümmten freien Endes (52) größer ist als ein Radius der Vertiefungen (48) im Bereich der Oberfläche (39) des Kopfs (30).

12. Vorrichtung (2) nach einem oder mehreren der vorstehenden Ansprüche, **dadurch gekennzeichnet, dass** das zweikomponentige Aufnahmeteil (24) vorzugsweise in jeder der Komponenten (26, 28) nach innen ausmündene Spülkanäle (44) aufweist, um einen Zwischenraum zwischen Aufnahmeteil (24) und Kopf (30) durch Einleitung von Spülflüssigkeit außerhalb der OP-Situation reinigen zu können.
